# EUROPÄISCHE PATENTSCHRIFT

(11) **EP 2 340 134 B1**
(45) Veröffentlichungstag und Bekanntmachung des Hinweises auf die Patenterteilung: **20.06.2012**
(21) Anmeldenummer: 09783834.6
(22) Anmeldetag: 08.10.2009
(51) Int. Cl.: B21D 39/04, B21D 51/26, B23P 11/00

(54) **VERFAHREN UND WERKZEUG ZUR ZYLINDRISCHEN VERFORMUNG EINER AL-HÜLSE AUF DAS KERNMASS DES INNENLIEGENDEN KUNSTSTOFFVERSCHLUSSES, ALS VORBEREITUNG EINER DIFFUSIONSDICHTEN PRESSVERBINDUNG INNERHALB DER BEIDEN BAUTEILE**
METHOD AND TOOL FOR THE CYLINDRICAL DEFORMATION OF AN AL SLEEVE TO THE CORE DIMENSION OF THE INTERNAL PLASTIC CLOSURE, AS PREPARATION FOR A DIFFUSION-PROOF PRESS CONNECTION WITHIN THE TWO COMPONENTS
PROCÉDÉ ET OUTIL DE FORMAGE CYLINDRIQUE D'UN MANCHON D'ALUMINIUM À LA DIMENSION DU NOYAU DU BOUCHON EN MATIÈRE PLASTIQUE PLACÉ À L'INTÉRIEUR, AFIN DE PRÉPARER UN ASSEMBLAGE PAR SERRAGE ÉTANCHE À LA DIFFUSION ENTRE LES DEUX PIÈCES

(30) Priorität: 09.10.2008 EP 08166264
(43) Veröffentlichungstag der Anmeldung: 06.07.2011
(73) Patentinhaber: Boehringer Ingelheim International GmbH, 55216 Ingelheim am Rhein (DE)
(72) Erfinder: SPREITZER, Joerg, 55216 Ingelheim am Rhein (DE); HAHN, Christoph, 55216 Ingelheim am Rhein (DE); HUEBNER, Michael, 55216 Ingelheim am Rhein (DE); MATHE, Gerald, 55216 Ingelheim am Rhein (DE); FUNKE, Peter, 15732 Eichwalde (DE); PUSCH, Ruediger, 15732 Eichwalde (DE)
(74) Vertreter: Hammann, Heinz
(86) Internationale Anmeldenummer: PCT/EP2009/063063
(87) Internationale Veröffentlichungsnummer: WO 2010/040791

(56) Entgegenhaltungen:
- WO-A2-00/49988
- DE-A1- 19 746 018
- DE-U1- 29 500 338

## Beschreibung

Die Erfindung bezieht sich auf ein Verfahren zur Herstellung einer Verbindung zwischen dem Halsbereich einer Leichtmetallkartusche, insbesondere einer Aluminiumkartusche, und einem darin eingesetzten Kunststoffbehälter durch einen einen Verbindungsbereich ausformenden und die Wand der Leichtmetallkartusche bereichsweise an den Kunststoffbehälter anlegenden Ziehprozess mit einem rotierenden Grundkörper eines Ziehwerkzeuges, der mindestens eine um eine Rotationsachse drehbare profilierte Ziehrolle zur Umgrenzung einer Werkzeugarbeitsöffnung lagert, wobei sich die Ziehrolle an die Mantelfläche der Leichtmetallkartusche anlegt.

Des Weiteren bezieht sich die Erfindung auf ein Ziehwerkzeug, das einen rotierbaren Grundkörper mit mindestens einer auf einer Kreisbahn angeordneten und um eine Rotationsachse drehbaren, profilierten Ziehrolle umfasst, wobei die Ziehrolle eine Werkzeugarbeitsöffnung abgrenzt und aus einer eine minimale Werkzeugarbeitsöffnung definierenden Position, den Durchmesser der Werkzeugarbeitsöffnung vergrö-βernd, gegen die Kraft einer Feder in eine maximale Werkzeugarbeitsöffnung definierende Position bewegbar ist.

Für die Applikation inhalationsfähiger pharmazeutischer Wirkstoffformulierungen finden vom Patienten in der Hand zu haltende und von Hand zu betätigende Inhalatoren Verwendung, in denen die pharmazeutische Wirkstoffformulierung jeweils in einer Inhalatorkartusche enthalten ist, die nach dem Verbrauch der Wirkstoffformulierung dem Inhalator entnommen und gegen eine neue Inhalatorkartusche ersetzt werden kann. Die Inhalatorkartusche besteht aus einer außenseitigen Aluminiumkartusche und einem darin eingesetzten Kunststoffbehälter, bei dem es sich häufig um einen im Koextrusionsverfahren hergestellten Kunststoffbehälter handelt, der einen steifen Außenbehälter und einen darin angeordneten flexiblen Innenbeutel umfasst. Um zwischen dem Innenbeutel und dem Außenbehälter eine Druckausgleichsöffnung zu schaffen, wird beispielsweise mit dem so genannten Cut-Crack-Open Prozess eine Öffnung in den relativ steifen Au-βenbehälter eingebracht. Der Kunststoffbehälter wird mit der pharmazeutischen Wirkstoffformulierung gefüllt und verschlossen und im Wege des Herstellungsprozesses zur Ausbildung der Inhalatorkartusche in die Aluminiumkartusche eingesetzt. In einem folgenden Schritt wird dann im oberen Bereich der derart zusammengesetzten Inhalatorkartusche ein verformter Wandbereich in der Aluminiumkartusche ausgebildet, der sich an die Außenfläche des in die Aluminiumkartusche eingesetzten Kunststoffbehälters anlegt. Zur Ausbildung des Verbindungsbereiches am Oberrand der Aluminiumkartusche wird ein Ziehverfahren angewendet, bei dem ein rotierendes Ziehwerkzeug mit seiner Werkzeugarbeitsöffnung von oben über den auszubildenden Verbindungsbereich der Aluminiumkartusche fährt und dabei rund um die Werkzeugarbeitsöffnung herum angeordnete, profilierte Ziehrollen in Anlage mit der Mantelfläche der Aluminiumkartusche bringt, um durch eine Axialbewegung den Verbindungsbereich ausformen. Anschließend wird die Aluminiumkartusche in weiteren Fertigungsschritten gasdicht an die Außenseite des darin angeordneten Kunststoffbehälters anlegt. Anschließend wird die Kombination von Aluminiumkartusche mit darin eingesetztem Kunststoffbehälter weiteren Prozess- und Verarbeitungsstufen zugeführt, an deren Ende dann die fertige, mit pharmazeutischer Wirkstoffformulierung befüllte Inhalatorkartusche steht.

Für die Durchführung des Ziehprozesses sind aus der Praxis verschiedene Ziehwerkzeuge bekannt. So gibt es Ziehwerkzeuge, bei welchen die profilierten Ziehrollen zur Ausbildung des profilierten Verbindungsbereiches weggesteuert ausgebildet sind. Aufgrund der sich bei der Fertigung von Aluminiumkartuschen und Kunststoffbehältern ergebenden Fertigungstoleranzen lässt sich mit derartigen Ziehwerkzeugen aber kein dauerhaft zufrieden stellendes Ergebnis erzielen. Es wurden daher auch Ziehwerkzeuge entwickelt, bei denen die Ziehrollen kraftgesteuert geführt und bewegt werden. Mit derartigen Ziehwerkzeugen lassen sich im Dauerbetrieb aber derzeit nur rund ca. 20000 Inhalatorkartuschen herstellen.

Bei einem aus der Praxis bekannten kraftgesteuerten Ziehwerkzeug ist außenseitig ein federkraftbeaufschlagter Kipphebel parallel zur Längsachse des Ziehwerkzeuges und um eine senkrecht zur Längsachse des Ziehwerkzeuges angeordnete Schwenkachse verschwenkbar angeordnet. Auf der der Feder abgewandten Seite liegt der Kipphebel an einem eine Ziehrolle tragenden Schieber an. Aufgrund der auf den Kipphebel einwirkenden Federkraft wird bei in die Werkzeugarbeitsöffnung eingeführter Aluminiumkartusche diese und jede weitere auf die gleiche Art ausgebildete Ziehrolle des Ziehwerkzeuges gegen die Mantelfläche der Aluminiumkartusche gedrückt. Hierdurch ist es möglich, dass beim Einführen der Aluminiumkartusche in die Werkzeugarbeitsöffnung die Ziehrollen zunächst gegen die Kraft der an dem Kipphebel angreifenden Feder nach außen ausweichen.

Im Fertigungsprozess mit diesem Ziehwerkzeug werden die still stehende Aluminiumkartusche und das rotierende Ziehwerkzeug mit gleicher Geschwindigkeit zunächst synchron derart übereinander geführt, dass sich die Aluminiumkartusche unterhalb der Werkzeugarbeitsöffnung des Ziehwerkzeuges befindet. Anschließend wird das Ziehwerkzeug dann von oben rotierend auf die Aluminiumkartusche abgesenkt, wodurch die die Werkzeugarbeitsöffnung umgebenden Ziehrollen mit der Mantelfläche der Aluminiumkartusche in Kontakt kommen und hierdurch ebenfalls in Rotationsbewegung versetzt werden. Das Ziehwerkzeug wird nun weiter abgesenkt, bis die Ziehrollen die zur Ausbildung des Verbindungsbereiches zu verformende Mantelfläche vollständig erfasst haben. Durch diese Absenkbewegung wird durch die profilierten Ziehrollen der Ziehprozess durchgeführt und dabei die Wandung der Aluminiumkartusche in dem Verbindungsbereich derart verformt, dass sich die Innenseite der Wandung der Aluminiumkartusche an die Außenfläche des darin eingesetzten Kunststoffbehälters anlegt. Um eine gleichmäßige Wanddicke der Aluminiumkartusche zu erzielen und eine Beschädigung an dem Kunststoffbehälter oder der Aluminiumkartusche auszuschließen, können die Ziehrollen, wie oben stehend beschrieben, weggesteuert radial gegen die Kraft der an dem Kipphebel angreifenden Druckfeder nach außen ausweichen. Die in dem Ziehwerkzeug eingestellte minimale Werkzeugarbeitsöffnung wird dabei auf ein Untermaß von ca. 0,2 mm eingestellt, so dass bei Einführung einer zu bearbeitenden Aluminiumkartusche die Ziehrollen immer auf Vorspannung stehen. Mit diesem kraftgesteuerten Werkzeug konnten in der Praxis aufgrund eines fehlenden Toleranzausgleichs noch keine vollständig zufrieden stellenden Ergebnisse erzielt werden.

Bei den weggesteuerten Ziehwerkzeugen besteht die Problematik, dass diese nicht sicher in der Lage sind, die Toleranzen der einzelnen Bauteile einer Inhalatorkartusche, wie den Kunststoffinnenbehälter, den Kunststoffverschluss des Kunststoffinnenbehälters, die Aluminiumkartusche sowie eine anzubringende Silikondichtung, auszugleichen. Als Folge davon kommt es beispielsweise bei vorhandenem Toleranzuntermaß zu so genannten Rattermarken am Hals des ausgebildeten Verformungsbereiches der Aluminiumkartusche. Bei Toleranz-übermaß, z. B. einem zu großen Verschluss des Kunststoffinnenbehälters oder einer Aluminiumkartusche mit zu kleinem Durchmesser, wird das beim Ziehprozess verformte und verdrängte Material nach oben gepresst und verlängert den Hals der Aluminiumkartusche in ungewünschter Weise. Beim nachfolgenden Bearbeitungsschritt der Inhalatorkartusche, dem Bördeln, entstehen dann unschöne Kanten oder gar so genannte Brauen.

Der Erfindung liegt die Aufgabe zugrunde, ein Verfahren und ein Ziehwerkzeug der eingangs genannten Art zu schaffen, mit denen einerseits Maßtoleranzabweichungen kompensiert und andererseits für einen Dauerbetrieb erforderliche Werkzeugstandzeiten erreicht werden.

Ausgehend von dem gattungsgemäßen Verfahren wird die Aufgabe erfindungsgemäß dadurch gelöst, dass die Rotationsachse der Ziehrolle an einem werkzeuginnenseitigen Endbereich eines quer zur Längsachse des Ziehwerkzeuges angeordneten Hebels angeordnet ist, der an seinem gegenüberliegenden werkzeugaußenseitigen Endbereich um eine Schwenkachse kraftgesteuert verschwenkbar und die Ziehrolle an die Mantelfläche der Leichtmetallkartusche anlegend an dem Ziehwerkzeug gelagert ist.

Die Aufgabe wird ausgehend von einem gattungsgemäßen Ziehwerkzeug erfindungsgemäß dadurch gelöst, dass die Rotationsachse der Ziehrolle an einem werkzeuginnenseitigen Endbereich eines quer zur Werkzeuglängsachse des Ziehwerkzeugs ausgerichteten Hebels angeordnet ist, der an seinem gegenüberliegenden werkzeugau-βenseitigen Endbereich um eine Schwenkachse kraftgesteuert verschwenkbar und die jeweilige Ziehrolle an die Mantelfläche des in die Werkzeugarbeitsöffnung eingeführten Werkstücks anlegend an dem Ziehwerkzeug gelagert ist.

Vorteilhafte und zweckmäßige Weiterbildungen und Ausgestaltungen der Erfindung ergeben sich aus den jeweiligen Unteransprüchen.

Sowohl das Verfahren als auch das Ziehwerkzeug zeichnen sich durch die Ausbildung eines quer zur Längsachse des Ziehwerkzeuges und quer zur Längsachse des die Werkzeugarbeitsöffnung ausbildenden Arbeitsbereiches des Ziehwerkzeuges angeordneten federkraftbeaufschlagten Hebels aus. An seinem werkzeuginnenseitigen Ende ist dem Hebel jeweils die Rotationsachse einer drehbaren Ziehrolle zugeordnet, wobei die Rotationsachse um eine Schwenkachse verlagerbar ist. In seinem werkzeugaußenseitigen Endbereich ist der Hebel um eine weitere Schwenkachse verschwenkbar gelagert, wobei in diesem werkzeugaußenseitigen Endbereich eine durch die Feder induzierte Kraft angreift. Aufgrund dieser Ausgestaltung kann die Ziehrolle einerseits gegen die Federkraft unter Erweiterung des Querschnitts der Werkzeugarbeitsöffnung von dem Hebel getragen eine Ausschwenk- oder Ausweichbewegung ausführen und andererseits wird die Ziehrolle durch die induzierte Kraft im Wege einer Einschwenk- oder Einfahrbewegung zur kraftgesteuerten Ausführung eines Ziehprozesses an die außenseitige Mantelfläche des in die Werkzeugarbeitsöffnung eingeführten Werkstückes angelegt und geführt. Auf diese Weise lässt sich in fertigungstechnisch zufrieden stellender Weise in einem Produktionsschritt der Herstellung einer Inhalatorkartusche der obere Randbereich der Aluminiumkartusche unter ziehender Verformung an die Außenseite des in der Aluminiumkartusche bereits angeordneten Kunststoffbehälters anlegen und dadurch in diesem Bereich eine Verbindung zwischen der Innenseite der Aluminiumkartusche und der Außenseite des Kunststoffbehälters ausbilden. Die Standzeiten sind bei der Verwendung des erfindungsgemäßen Ziehwerkzeuges dadurch verbessert, dass die jeweilige Ziehrolle, und vorteilhafterweise alle Ziehrollen, nun nicht mehr eine schiebergesteuerte lediglich translatorische Bewegung in radialer Richtung der Werkzeugarbeitsöffnung ausführt, sondern aufgrund ihrer Anordnung an dem schwenkbaren Hebel eine Bewegung in radialer Richtung. Aufgrund dieser Bewegung erfolgt die Ausweichbewegung aus dem und die Einfahrbewegung in den Bereich der Werkzeugarbeitsöffnung sowohl für die jeweilige Ziehrolle als auch das zu bearbeitende Werkstück deutlich sanfter. Der quer zur Längsachse der Werkzeugsarbeitsöffnung und/oder des Ziehwerkzeuges angeordnete Hebel ist vorzugsweise im Wesentlichen senkrecht zu dieser Längsachse, d. h. bei im Betrieb befindlichem Ziehwerkzeug im Wesentlichen waagerecht ausgerichtet.

Um das kraftgesteuerte Anlegen der jeweiligen Ziehrolle an das zu bearbeitende Werkstück sicherzustellen, greift vorzugsweise an dem werkzeugaußenseitigen Endbereich des Hebels eine durch eine Feder induzierte Kraft an und der Verbindungsbereich durch Verschwenken des Hebels unter Ausbildung einer einen Schwenkwinkel überstreichenden Schwenkbewegung und ein dadurch bewirktes kraftgesteuertes Anlegen der Ziehrolle an die Mantelfläche der Leichtmetallkartusche ausgebildet wird, wobei die Ziehrolle aufgrund einer weiteren Schwenkachse eine Axialbewegung ausführt.

Da das Verfahren vorzugsweise für die Herstellung einer Inhalatorkartusche zur Verwendung in einem Inhalator zur Applikation von Wirkstoffformulierungen durch einen Patienten selbst zur Anwendung kommt, wird zweckmäßigerweise der Kunststoffbehälter in einem oder mehreren anschließenden Verfahrensschritten gasdicht mit der Leichtmetallkartusche verbunden und mit einer pharmazeutischen Wirkstoffformulierung befüllt sowie verschlossen. Vorzugsweise werden die Leichtmetallkartusche und der Kunststoffbehälter anschlie-βend zu einer Inhalatorkartusche für die Verwendung in einem Inhalator weiterverarbeitet.

In Ausgestaltung des Ziehwerkzeuges greift an dem werkzeugaußenseitigen Endbereich des Hebels eine durch die Feder induzierte Kraft an, gegen die der Hebel derart um die Schwenkachse verschwenkbar ist, dass die Rotationsachse aus der der minimalen Werkzeugarbeitsöffnung zugeordneten Position der Ziehrolle heraus und wieder in diese Position hinein bewegbar ist.

Um mit Hilfe lediglich einer Feder den Hebel und damit die daran angeordnete Ziehrolle kraftgesteuert zu beaufschlagen, greift der werkzeugaußenseitige Bereich des Hebels an einer zumindest bereichsweise drehbar an dem Grundkörper befestigten Ringscheibe an, an der die Feder vorgespannt angeordnet ist, wobei der werkzeugaußenseitige Bereich bei der radialen Ausweichbewegung der Ziehrolle die Ringscheibe bereichsweise gegen die Kraft der Feder verdrehbar und die Einfahr- bzw. Einschubbewegung der Ziehrolle durch ein von der Federkraft getriebenes Zurückdrehen der Ringscheibe mit von dieser in den werkzeugaußenseitigen Bereich des Hebels eingeleiteter Kraftübertragung bewirkbar ist. Die Ringscheibe bietet besondere Vorteile bei der Anordnung mehrerer Ziehrollen an jeweils einem Hebel, da in dieser Ausgestaltung lediglich eine Feder benötigt wird und bei einem Klemmen eines Hebels dieser über die anderen der Ringscheibe zugeordneten Hebel zwangsweise verlagert wird. Im Weiteren wirkt auf sämtliche Ziehrollen dieselbe Kraft.

Um beim Einstellen bzw. Justieren des Ziehwerkzeuges radiales Spiel zwischen dem Hebel und der Ringscheibe eliminieren zu können, ist die Schwenkachse im werkzeugaußenseitige Bereich des Hebels im Zentrum einer exzentrisch gelagerten Hülse gebildet, die an der Ringscheibe angreift. Die Hülse wirkt in der rotierenden Wirkrichtung des Ziehwerkzeuges insbesondere spielfrei mit der Ringscheibe zusammen, um ein fehlerfreies Werkstück reproduzierbar zu fertigen. Im Weiteren dient die Schwenkachse zur verschwenkbaren Lagerung der entsprechenden Schwenkachse des Hebels.

Um eine relativ geradlinige Ausweichbewegung der Ziehrolle zum Toleranzausgleich zu erzeugen, ist eine weitere Schwenkachse im Bereich der Ziehrolle gebildet. Zur Bereitstellung günstiger Hebelverhältnisse und zur Realisierung der für den Toleranzausgleich notwendigen Bewegungen sind zweckmäßigerweise die beiden Schwenkachsen in der Längsachse des Hebels und der Mittelpunkt der Rotationsachse seitlich versetzt dazu ausgerichtet, wobei die Schwenkachse im Bereich der Ziehrolle um insbesondere 1 mm zu der Rotationsachse versetzt ist.

Der Abstand zwischen der weiteren Schwenkachse im Bereich der Ziehrolle und der Rotationsachse bildet einen Hebelarm aus. Vorzugsweise besteht zwischen dem Hebelarm und dem Hebel ein Hebelverhältnis von 1 : 28.

Nach einer Weiterbildung haltert die weitere Schwenkachse die Ziehrolle exzentrisch verstellbar und ist in Lagerflanschen eines werkzeugfesten Lagerbocks drehbar gelagert sowie an dem Hebel drehfest angeordnet. Durch eine Verstellung des Lagerbocks wird eine Justage des Ziehwerkzeugs vorgenommen, um die Größe der Werkzeugarbeitsöffnung und damit des Außendurchmesser des Verbindungsbereichs zwischen der zylindrischen Aluminiumkartusche und dem darin eingesetzten Kunststoffbehälter festzulegen.

Zweckmäßigerweise ermöglicht die radiale Ausweichbewegung der Ziehrolle einen Rollenhub von 0,3 mm und der Hebel überstreicht dabei einen Schwenkwinkel von 22°.

Um insbesondere bei kreiszylindrischen Leichtmetallkartuschen eine exakte Ausformung des bearbeiteten Wandbereiches zu erzielen, sind bevorzugt an dem Grundkörper mindestens drei Ziehrollen, vorzugsweise fünf Ziehrollen, angeordnet sind, die gleichmäßig zueinander beabstandet die Werkzeugarbeitsöffnung umgrenzen.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird nachfolgend anhand eines Ausführungsbeispieles unter Bezugnahme auf die zugehörigen Zeichnungen näher erläutert. Es zeigt:

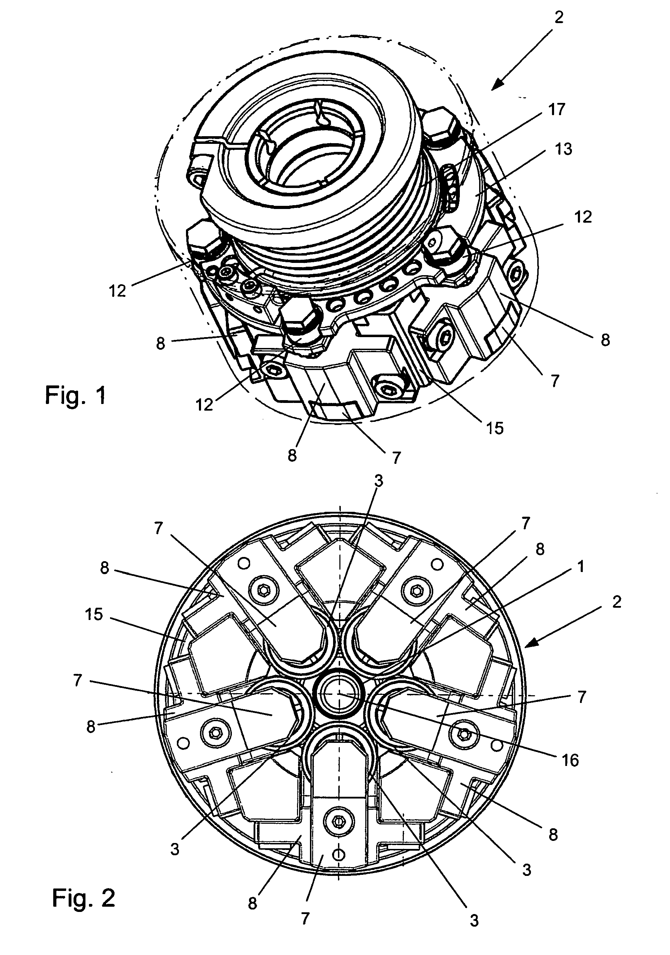

- Fig. 1: eine perspektivische Darstellung des Ziehwerkzeuges,
- Fig. 2: eine Darstellung des Ziehwerkzeuges nach Fig. 1 von unten,

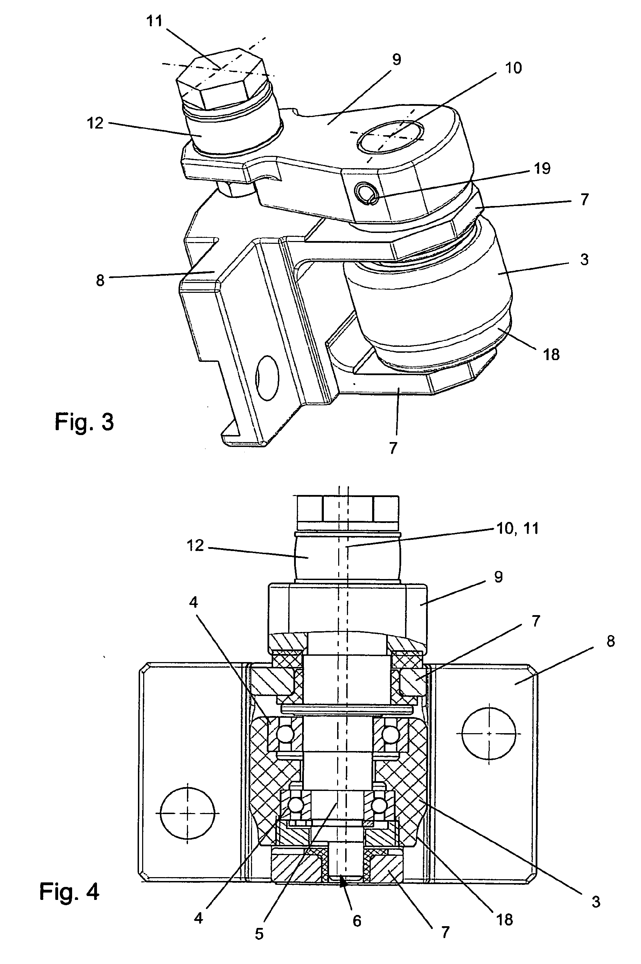

- Fig. 3: eine Perspektivdarstellung einer Rolleneinheit des Ziehwerkzeuges
- Fig. 4: einen Querschnitt durch die Rolleneinheit nach Fig. 3 und

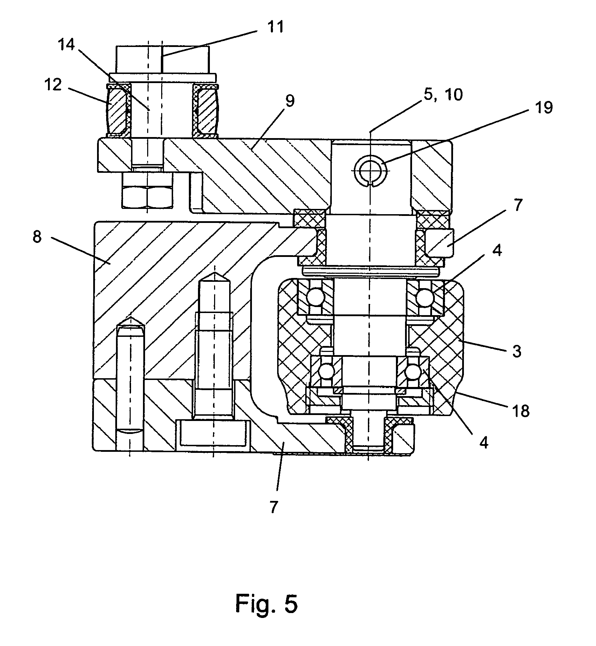

- Fig. 5: einen Längsschnitt durch die Rolleneinheit nach Fig. 3.

Zur Durchführung eines Ziehprozesses ist im Zentrum eines rotierbaren Ziehwerkzeuges 2 innenseitig eine Werkzeugarbeitsöffnung 1 ausgebildet, die von fünf die Werkzeugarbeitsöffnung 1 auf einer Kreisbahn umgebenden Ziehrollen 3gebildet ist. Nachstehend ist lediglich eine der Ziehrollen 3 näher beschrieben, da alle Ziehrollen 3 identisch aufgebaut sind.

Die Ziehrolle 3 ist mittels zwei Kugellager 4 auf einer eine Rotationsachse 5 bildenden Exzenterachse 6 drehbar befestigt, wobei die Exzenterachse 6 exzentrisch verdrehbar in zwei die Ziehrolle 3 zwischen sich aufnehmenden Lagerflanschen 7 eines Lagerbock 8 des Ziehwerkzeuges 2 aufgenommen ist. Ein über den Lagerbock 8 hervorstehendes Ende der Exzenterachse 6 ist mittels eines Querstiftes 19 drehfest an einem werkzeuginnenseitigen Endbereich eines Hebels 9 angeordnet. Die zu der Rotationsachse 5 seitlich versetzte Mittenachse der Exzenterachse 6 bildet eine Schwenkachse 10. An seinem der Ziehrolle 3 gegenüberliegenden werkzeugaußenseitigen Endbereich ist der Hebel 9 um eine weitere Schwenkachse 11 mittels einer diese umgebenden Hülse 12 verschwenkbar an einer Ringscheibe 13 gehalten. Die beiden Schwenkachsen 10, 11 fluchten in ihrer Normalstellung und die Hülse 12 ist mittels eines Exzenters 14 verstellbar an dem Hebel 9 angeordnet.

Die Ringscheibe 13 ist an einem Grundkörper 15 des Ziehwerkzeuges 2 um dessen Längsachse 16 gegen die Kraft einer als Ringfeder ausgebildeten Feder 17 drehbar gelagert. Das eine Ende der Feder 17 greift hierbei in die Ringscheibe 13 ein und das andere Ende der Feder 17 ist an dem Grundkörper 15 des Ziehwerkzeuges 2 festgelegt. Der Hebel 9 ist quer zur Längsachse 16 des Ziehwerkzeugs 1 ausgerichtet.

Zum Justieren des Ziehwerkzeuges 2 wird mit einem Lehrdorn die Werkzeugarbeitsöffnung 1 auf einen Durchmesser eingestellt, der dem gewünschten Außendurchmesser einer Leichtmetallkartusche. Hierbei werden die Lagerböcke 8 verschoben und die Rotationsachsen 5 der Ziehrollen 3 sind seitlich versetzt ausgerichtet. Durch ein Verdrehen der Exzenters 14 werden die Hülsen 12 zur Anlage in entsprechenden Ausnehmungen der Ringscheibe 13 gebracht, wobei die Ausnehmungen die Hülsen 12 mit Spiel aufnehmen.

Im Betriebszustand rotiert das Ziehwerkzeug 2 und in die Werkzeugarbeitsöffnung 1 wird ein Werkstück, also die Leichtmetallkartusche mit einem darin eingesetzten Kunststoffbehälter, eingebracht. Das Ziehwerkzeug 2 verfährt in axialer Richtung nach unten auf das Werkstück und die Einlaufschrägen 18 der Ziehrollen 3 kommen in Eingriff mit dem Werkstück, worauf die Ziehrollen um Ihre Rotationsachsen drehen. Bei einer weiteren nach unten gerichteten Axialbewegung wird das Werkstück durch die Ziehrollen 3 verjüngt und es wird durch die die Einlaufschrägen 18 eine Schulter ausgeformt.

Damit das Werkstück nicht beschädigt und dennoch eine zufrieden stellende Verbindung zwischen dem Halsbereich der Leichtmetallkartusche und dem darin eingesetzten Kunststoffbehälter erzielt wird, führt jeder Hebel 9 eine Schwenkbewegung gegen die Kraft der Feder 17 aus, die zu im Wesentlichen radialen Bewegungen der Ziehrollen 3 führen, um den Durchmesser der Werkzeugarbeitsöffnung 1 zu vergrö-βern. Die entsprechenden Ausweichbewegungen erfolgen über die Schwenkachsen 10, 11 des Hebels 9 im Bereich der verdrehten Ringscheibe 13 und der rotierenden Ziehrolle 3. Die Ziehrollen 3 bleiben so lange federbelastet in der Bearbeitungsposition, bis das Ziehwerkzeug 2 in einer Axialbewegung außer Eingriff mit dem Werkstück gebracht wird. Sonach stellen sich die Ziehrollen 3 aufgrund der Wirkung der Feder 17 in ihre justierte Position. Hierbei wird die Ringscheibe 13 durch die Kraft der Feder 17 verdreht und dabei werden die Hebel 9 verschwenkt, um die Ziehrollen 3 zu verlagern.

Im Ausführungsbeispiel sind fünf Ziehrollen 3 dargestellt. Das Ziehwerkzeug 2 kann aber auch eine beliebige andere Anzahl an Ziehrollen 3, insbesondere auch eine oder drei Ziehrollen 3 aufweisen.

Der sich zwischen der an der Ringscheibe 11 anliegenden Hülse 10 und der der Ziehrolle 3 zugeordneten Schwenkachse 10 erstreckende Hebel 8 steht in einem Verhältnis von 1:28 zu dem Hebelarm, der zwischen der Rotationsachse 5 der Ziehrolle 3 und der ihr zugeordneten Schwenkachse 10 gebildet ist.

Der Mittelpunkt der Schwenkachse 10 und der Mittelpunkt der Rotationsachse 5 sind seitlich versetzt zueinander an dem Hebel 9 angeordnet, wobei vorzugsweise der Mittelpunkt der Rotationsachse 5 um insbesondere 1 mm außerhalb der Längsachse des Hebels 9 und der Mittelpunkt der Schwenkachse 10 auf der Längsachse des Hebels 9 angeordnet ist. Die Ausschwenkbewegung des Hebels 9 ermöglicht jeweils einen Rollenhub der Ziehrollen 3 von 0,3 mm und überstreicht dabei einen Schwenkwinkel von 22°.

## Patentansprüche

1. Verfahren zur Herstellung einer Verbindung zwischen dem Halsbereich einer Leichtmetallkartusche, insbesondere einer Aluminiumkartusche, und einem darin eingesetzten Kunststoffbehälter durch einen Verbindungsbereich ausformenden und die Wand der Leichtmetallkartusche bereichsweise an den Kunststoffbehälter anlegenden Ziehprozess mit einem rotierenden Grundkörper (15) eines Ziehwerkzeuges (2), der mindestens eine um eine Rotationsachse (5) drehbare profilierte Ziehrolle (3) zur Umgrenzung einer Werkzeugarbeitsöffnung (1) lagert, wobei sich die Ziehrolle (3) an die Mantelfläche der Leichtmetallkartusche anlegt, **dadurch gekennzeichnet, dass** die Rotationsachse (5) der Ziehrolle (3) an einem werkzeuginnenseitigen Endbereich eines quer zur Längsachse (16) des Ziehwerkzeuges (2) angeordneten Hebels (9) angeordnet ist, der an seinem gegenüberliegenden werkzeugaußenseitigen Endbereich um eine Schwenkachse (11) kraftgesteuert verschwenkbar und die Ziehrolle (3) an die Mantelfläche der Leichtmetallkartusche anlegend an dem Ziehwerkzeug (2) gelagert ist.

2. Verfahren nach Anspruch 1, **dadurch gekennzeichnet, dass** an dem werkzeugaußenseitigen Endbereich des Hebels (9) eine durch eine Feder (17) induzierte Kraft angreift und der Verbindungsbereich durch Verschwenken des Hebels (9) unter Ausbildung einer einen Schwenkwinkel überstreichenden Schwenkbewegung und ein dadurch bewirktes kraftgesteuertes Anlegen der Ziehrolle (3) an die Mantelfläche der Leichtmetallkartusche ausgebildet wird, wobei die Ziehrolle (3) aufgrund einer weiteren Schwenkachse (10) eine Axialbewegung ausführt.

3. Verfahren nach Anspruch 1 oder 2, **dadurch gekennzeichnet, dass** es mit einem Ziehwerkzeug (2) nach einem der Ansprüche 5 bis 14 durchgeführt wird.

4. Verfahren nach einem der Ansprüche 1 bis 3, **dadurch gekennzeichnet, dass** der Kunststoffbehälter in einem oder mehreren anschließenden Verfahrensschritten gasdicht mit der Leichtmetallkartusche verbunden und mit einer pharmazeutischen Wirkstoffformulierung befüllt sowie verschlossen wird und/oder die Leichtmetallkartusche und der Kunststoffbehälter anschließend zu einer Inhalatorkartusche für die Verwendung in einem Inhalator weiterverarbeitet werden..

5. Ziehwerkzeug, das einen rotierbaren Grundkörper (15) mit mindestens einer auf einer Kreisbahn angeordneten und um eine Rotationsachse (5) drehbaren, profilierten Ziehrolle (3) umfasst, wobei die Ziehrolle (3) eine Werkzeugarbeitsöffnung (1) abgrenzt und aus einer eine minimale Werkzeugarbeitsöffnung (1) definierenden Position, den Durchmesser der Werkzeugarbeitsöffnung (1) vergrößernd, gegen die Kraft einer Feder (17) in eine maximale Werkzeugarbeitsöffnung definierende Position bewegbar ist, **dadurch gekennzeichnet, dass** die Rotationsachse (5) der Ziehrolle (3) an einem werkzeuginnenseitigen Endbereich eines quer zur Werkzeuglängsachse (16) des Ziehwerkzeugs (2) ausgerichteten Hebels (9) angeordnet ist, der an seinem gegenüberliegenden werkzeugaußenseitigen Endbereich um eine Schwenkachse (11) kraftgesteuert verschwenkbar und die Ziehrolle (3) an die Mantelfläche des in die Werkzeugarbeitsöffnung (1) eingeführten Werkstücks anlegend an dem Ziehwerkzeug (2) gelagert ist.

6. Ziehwerkzeug nach Anspruch 5, **dadurch gekennzeichnet, dass** an dem werkzeugaußenseitigen Endbereich des Hebels (9) eine durch die Feder (17) induzierte Kraft angreift, gegen die der Hebel (8) derart um die Schwenkachse (11) verschwenkbar ist, dass die Rotationsachse (9) aus der der minimalen Werkzeugarbeitsöffnung (1) zugeordneten Position der Ziehrolle (3) heraus und wieder in diese Position hinein bewegbar ist.

7. Ziehwerkzeug nach Anspruch 5 oder 6, **dadurch gekennzeichnet, dass** der werkzeugaußenseitige Bereich des Hebels (9) an einer zumindest bereichsweise drehbar an dem Grundkörper (15) befestigten Ringscheibe (13) angreift, an der die Feder (17) vorgespannt angeordnet ist, wobei der werkzeugaußenseitige Bereich bei der radialen Ausweichbewegung der Ziehrolle (3) die Ringscheibe (13) bereichsweise gegen die Kraft der Feder (17) verdrehbar und die Einschubbewegung der Ziehrolle (3) durch ein von der Federkraft getriebenes Zurückdrehen der Ringscheibe (13) mit von dieser in den werkzeugaußenseitigen Bereich des Hebels (9) eingeleiteter Kraftübertragung bewirkbar ist.

8. Ziehwerkzeug nach einem der Ansprüche 5 bis 7, **dadurch gekennzeichnet, dass** die Schwenkachse (11) im werkzeugaußenseitige Bereich des Hebels (9) im Zentrum einer exzentrisch gelagerten Hülse (12) gebildet ist, die an der Ringscheibe (13) angreift.

9. Ziehwerkzeug nach einem der Ansprüche 5 bis 8, **dadurch gekennzeichnet, dass** eine weitere Schwenkachse (10) im Bereich der Ziehrolle (3) gebildet ist.

10. Ziehwerkzeug nach einem der Ansprüche 5 bis 9, **dadurch gekennzeichnet, dass** die beiden Schwenkachsen (10, 11) in der Längsachse des Hebels (9) und der Mittelpunkt der Rotationsachse (5) seitlich versetzt dazu ausgerichtet sind, wobei die Schwenkachse (10) im Bereich der Ziehrolle (3) um insbesondere 1 mm zu der Rotationsachse (5) versetzt ist.

11. Ziehwerkzeug nach Anspruch 10, **dadurch gekennzeichnet, dass** der Abstand zwischen der weiteren Schwenkachse (10) im Bereich der Ziehrolle (3) und der Rotationsachse (5) einen Hebelarm ausbildet.

12. Ziehwerkzeug nach Anspruch 11, **dadurch gekennzeichnet, dass** zwischen dem Hebelarm und dem Hebel (9) ein Hebelverhältnis von 1: 28 besteht.

13. Ziehwerkzeug nach einem der Ansprüche 10 bis 12, **dadurch gekennzeichnet, dass** die weitere Schwenkachse (10) die Ziehrolle (3) exzentrisch verstellbar haltert und in Lagerflanschen (7) eines werkzeugfesten Lagerbocks (8) drehbar gelagert sowie an dem Hebel (9) drehfest angeordnet ist.

14. Ziehwerkzeug nach einem der Ansprüche 5 bis 13, **dadurch gekennzeichnet, dass** die radiale Ausweichbewegung der Ziehrolle (3) einen Rollenhub von 0,3 mm ermöglicht und der Hebel (9) dabei einen Schwenkwinkel von 22° überstreicht.

15. Ziehwerkzeug nach einem der Ansprüche 5 bis 14, **dadurch gekennzeichnet, dass** an dem Grundkörper (12) mindestens drei Ziehrollen (3), vorzugsweise fünf Ziehrollen (3), angeordnet sind, die gleichmäßig zueinander beabstandet die Werkzeugarbeitsöffnung (1) umgrenzen.

## Claims

1. Method of producing a connection between the neck area of a light metal cartridge, particularly an aluminium cartridge, and a plastic container inserted therein, by a drawing process that forms a connection area and applies areas of the wall of the light metal cartridge against the plastic container using a rotating main body (15) of a drawing tool (2), said main body supporting at least one profiled drawing roller (3) which can rotate about a rotation axis (5) in order to delimit a tool working opening (1), wherein the drawing roller (3) lies against the outer surface of the light metal cartridge, **characterised in that**
the rotation axis (5) of the drawing roller (3) is arranged at a tool-internal end area of a lever (9) arranged transversely relative to the longitudinal axis (16) of the drawing tool (2), at its opposite tool-external end area the lever is supported on the drawing tool (2) so as to be pivotable about a pivot axis (11) in a force- controlled manner and so as to apply the drawing roller (3) against the outer surface of the light metal cartridge.

2. Method according to claim 1, **characterised in that** a force induced by a spring (17) acts on the tool-external end area of the lever (9) and the connection area is formed by pivoting the lever (9), thereby producing a pivoting movement covering a pivot angle, and a resultant force-contrelled application of the drawing roller (3) against the outer surface of the light metal cartridge, the drawing roller (3) performing an axial movement thanks to a further pivot axis (10).

3. Method according to Claim 1 or 2, **characterised in that** it is carried out with a drawing tool (2) according to one of claims 5 to 14.

4. Method according to one of claims 1 to 3, **characterised in that** the plastic container is connected in gastight manner to the light metal cartridge in one or more subsequent steps and is filled with a pharmaceutical active substance formulation and sealed, and/or the light metal cartridge and the plastic container are then further processed to form an inhaler cartridge for use in an inhaler.

5. Drawing tool which comprises a rotatable main body (15) having at least one profiled drawing roller (3) arranged on a circular track and rotatable about a rotation axis (5), wherein the drawing roller (3) delimits a tool working opening (1) and can be moved out of a position that defines a minimum tool working opening (1), enlarging the diameter of the tool working opening (1), counter to the force of a spring (17), into a position that defines a maximum tool working opening, **characterised in that** the rotation axis (5) of the drawing roller (3) is arranged on a tool-internal end area of a lever (9) aligned transversely with respect to the longitudinal axis (16) of the drawing tool (2), said lever (9) being supported at its opposite tool-external end area on the drawing tool (2) so as to be pivotable about a pivot axis (11) in a force controlled manner and so as to apply the drawing roller (3) against the outer surface of the workpiece introduced into the tool working opening (1).

6. Drawing tool according to Claim 5, **characterised in that** a force induced by the spring (17) acts on the tool-external end area of the lever (9), against which the lever (8) is pivotable about the pivot axis (11) such that the rotation axis (9) is able to move out of the position of the drawing roller (3) associated with the minimum tool working opening (1) and back into this position again.

7. Drawing tool according to Claim 5 or 6, **characterised in that** the tool-external region of the lever (9) acts on an annular disc (13) which is at least partly rotatably attached to the main body (15) and on which the spring (17) is arranged under pre-stress, wherein the tool-external region during the radial deflecting movement of the drawing roller (3) the annular disc (13) being rotatable in parts counter to the force of the spring (17) and the insertion movement of the drawing roller (3) being achievable by a back-rotation of the annular disc (13) propelled by spring force, with transmission of force being introduced therefrom into the tool-external region of the lever (9).

8. Drawing tool according to one of claims 5 to 7, **characterised in that** the pivot axis (11) is formed in the tool-external region of the lever (9) in the centre of an eccentrically mounted sleeve (12) which acts on the annular disc (13).

9. Drawing tool according to one of claims 5 to 8, **characterised in that** a further pivot axis (10) is formed in the region of the drawing roller (3).

10. Drawing tool according to one of claims 5 to 9, **characterised in that** the two pivot axes (10, 11) along the longitudinal axis of the lever (9) and the centre point of the rotation axis (5) are aligned laterally offset therefrom, the pivot axis (10) in the region of the drawing roller (3) being offset by, in particular, 1 mm from the rotation axis (5).

11. Drawing tool according to Claim 10, **characterised in that** the distance between the further pivot axis (10) in the region of the drawing roller (3) and the rotation axis (5) forms a lever arm.

12. Drawing tool according to Claim 11, **characterised in that** there is a lever ratio of 1 : 28 between the lever arm and the lever (9).

13. Drawing tool according to one of claims 10 to 12, **characterised in that** the further pivot axis (10) supports the drawing roller (3) in an eccentrically movable manner and is rotatably mounted in bearing flanges (7) of a bearing block attached to the tool and is mounted in rotation-locked manner on the lever (9).

14. Drawing tool according to one of claims 5 to 13, **characterised in that** the radial deflecting movement of the drawing roller (3) enables a roller travel of 0.3 mm and the lever (9) covers a pivot angle of 22°.

15. Drawing tool according to one of claims 5 to 14, **characterised in that** there are at least three drawing rollers (3), preferably five drawing rollers (3), arranged on the main body (12), at a uniform spacing from one another, delimiting the tool working opening (1).

## Revendications

1. Procédé de fabrication d'une liaison entre la zone de goulet d'une cartouche en métal léger, en particulier d'une cartouche en aluminium, et un récipient en matière plastique inséré dans cette dernière grâce à un procédé d'emboutissage formant une zone de liaison et appliquant la paroi de la cartouche en métal léger par endroits au niveau du récipient en matière plastique, avec un corps de base (15) rotatif d'un outil d'emboutissage (2), lequel corps de base loge au moins un galet d'emboutissage (3) profilé pouvant tourner autour d'un axe de rotation (5) en vue de délimiter une ouverture de travail d'outil (1), le galet d'emboutissage (3) s'appliquant contre l'enveloppe de la cartouche en métal léger, **caractérisé en ce que** l'axe de rotation (5) du galet d'emboutissage (3) est disposé au niveau d'une zone d'extrémité côté intérieur d'un outil de levier (9) disposé de manière transversale par rapport à l'axe longitudinal (16) de l'outil d'emboutissage (2), lequel outil de levier peut pivoter de manière commandée par une force autour d'un axe de pivotement (11), au niveau de sa zone d'extrémité opposée côté extérieur de l'outil, et **en ce que** le galet d'emboutissage (3) est logé contre l'outil d'emboutissage (2) de manière à s'appliquer contre l'enveloppe de la cartouche en métal léger.

2. Procédé selon la revendication 1, **caractérisé en ce qu'**une force induite par un ressort (17) agit sur la zone d'extrémité côté extérieur de l'outil de levier (9), et **en ce que** la zone de liaison est réalisée par pivotement de l'outil de levier (9) en réalisant un mouvement de pivotement balayant un angle de pivotement et en appliquant en conséquence de manière commandée par une force le galet d'emboutissage (3) contre l'enveloppe de la cartouche en métal léger, le galet d'emboutissage (3) exécutant un mouvement axial du fait d'un axe de pivotement supplémentaire (10).

3. Procédé selon la revendication 1 ou 2, **caractérisé en ce qu'**il est exécuté avec un outil d'emboutissage (2) selon l'une quelconque des revendications 5 à 14.

4. Procédé selon l'une quelconque des revendications 1 à 3, **caractérisé en ce que** le récipient en matière plastique est relié de manière étanche au gaz à la cartouche en métal léger au cours d'une ou de plusieurs étapes de procédé consécutives, est rempli d'une préparation de substances actives pharmaceutique et est fermé, et/ou en ce que la cartouche de métal léger et le récipient en matière plastique sont par la suite transformés en une cartouche d'inhalateur pour l'utilisation dans un inhalateur.

5. Outil d'emboutissage, qui comporte un corps de base (15) rotatif doté au moins d'un galet d'emboutissage (3) profilé, disposé sur une trajectoire circulaire et pouvant pivoter autour d'un axe de rotation (5), le galet d'emboutissage (3) délimitant une ouverture de travail d'outil (1) et pouvant être déplacé contre la force d'un ressort (17), d'une position définissant une ouverture de travail d'outil (1) minimale, agrandissant le diamètre de l'ouverture de travail de l'outil (1), dans une position définissant une ouverture de travail d'outil maximale, **caractérisée en ce que** l'axe de rotation (5) du galet d'emboutissage (3) est disposé au niveau d'une zone d'extrémité côté intérieur de l'outil de levier (9) orienté de manière transversale par rapport à l'axe longitudinal d'outil (16) de l'outil d'emboutissage (2), lequel outil de levier peut pivoter, au niveau de sa zone d'extrémité opposée côté extérieur de l'outil, autour d'un axe de pivotement (11) de manière commandée par une force, et **en ce que** le galet d'emboutissage (3) est logé au niveau de l'outil d'emboutissage (2) de manière à s'appliquer contre l'enveloppe de la pièce à usiner introduite dans l'ouverture de travail d'outil (1).

6. Outil d'emboutissage selon la revendication 5, **caractérisé en ce qu'**une force induite par le ressort (17) agit sur la zone d'extrémité côté extérieur de l'outil de levier (9), contre laquelle le levier (8) peut pivoter autour de l'axe de rotation (11) de telle manière que l'axe de rotation (9) peut être déplacé en dehors de la position du galet d'emboutissage (3) associée à l'ouverture de travail d'outil (1) minimale et peut être replacé à nouveau dans cette position.

7. Outil d'emboutissage selon la revendication 5 ou 6, **caractérisé en ce que** la zone côté extérieur de l'outil de levier (9) agit sur un disque annulaire (13) fixé au moins par endroits de manière rotative au niveau du corps de base (15), sur lequel disque annulaire le ressort (17) est disposé de manière précontrainte, la zone côté extérieur de l'outil amenant le disque annulaire (13) à pivoter par endroits contre la force du ressort (17) lors du déplacement alternatif radial du galet d'emboutissage (3) et le déplacement par insertion du galet d'emboutissage (3) pouvant être provoqué par une rotation inverse du disque annulaire (13), entraînée par la force de ressort avec la transmission d'une force introduite par le disque annulaire dans la zone du côté extérieur de l'outil de levier (9).

8. Outil d'emboutissage selon l'une quelconque des revendications 5 à 7, **caractérisé en ce que** l'axe de pivotement (11) est formé dans la zone côté extérieur de l'outil de levier (9), au centre d'un manchon (12) logé de manière excentrique, lequel manchon agit sur le disque annulaire (13).

9. Outil d'emboutissage selon l'une quelconque des revendications 5 à 8, **caractérisé en ce qu'**un axe de pivotement supplémentaire (10) est formé dans la zone du galet d'emboutissage (3).

10. Outil d'emboutissage selon l'une quelconque des revendications 5 à 9, **caractérisé en ce que** les deux axes de pivotement (10, 11) sont disposés dans l'axe longitudinal de l'outil de levier (9), et **en ce que** le point médian de l'axe de rotation (5) est orienté de manière décalée latéralement par rapport à ces derniers, l'axe de pivotement (10) étant décalé dans la zone du galet d'emboutissage (3) en particulier de 1 mm par rapport à l'axe de rotation (5).

11. Outil d'emboutissage selon la revendication 10, **caractérisé en ce que** la distance entre l'axe de pivotement supplémentaire (10) dans la zone du galet d'emboutissage (3) et l'axe de rotation (5) forme un bras de levier.

12. Outil d'emboutissage selon la revendication 11, **caractérisé en ce qu'**un rapport de levier entre le bras de levier et l'outil de levier (9) est de 1:28.

13. Outil d'emboutissage selon l'une quelconque des revendications 10 à 12, **caractérisé en ce que** l'axe de pivotement supplémentaire (10) maintient le galet d'emboutissage (3) de manière ajustable excentriquement, est logé de manière rotative dans des flasques de palier (7) d'un support de palier (8) solidaire de l'outil et est disposé de manière solidaire en rotation au niveau de l'outil de levier (9).

14. Outil d'emboutissage selon l'une quelconque des revendications 5 à 13, **caractérisé en ce que** le déplacement alternatif radial du galet d'emboutissage (3) permet un levage des galets de 0,3 mm, et **en ce que** l'outil de levier (9) balaye dans ce cadre un angle de pivotement de 22°.

15. Outil d'emboutissage selon l'une quelconque des revendications 5 à 14, **caractérisé en ce qu'**au moins trois galets d'emboutissage (3), de préférence cinq galets d'emboutissage (3), sont disposés au niveau du corps de base (12), lesquels espacées les uns des autres de manière homogène, délimitent l'ouverture de travail d'outil (1).
